# EUROPEAN PATENT SPECIFICATION

(11) **EP 4 136 595 B1**
(45) Date of publication and mention of the grant of the patent: **19.11.2025**
(21) Application number: 21788293.5
(22) Date of filing: 09.04.2021
(51) Int. Cl.: G16H 10/60, G06Q 10/00, G06Q 10/06, G06Q 50/00

(54) **SYSTEMS AND METHODS FOR VERIFYING PATIENT IMMUNITY**
SYSTEME UND VERFAHREN ZUR ÜBERPRÜFUNG DER IMMUNITÄT EINES PATIENTEN
SYSTÈMES ET PROCÉDÉS POUR VÉRIFIER UNE IMMUNITÉ DE PATIENT

(30) Priority: 13.04.2020 US 202063009133 P
(43) Date of publication of application: 22.02.2023
(73) Proprietor: Rajagopal, Dhiren, Atlanta, GA 30327 (US); Rajagopal, Deven, Atlanta, GA 30327 (US)
(72) Inventor: Rajagopal, Dhiren, Atlanta, GA 30327 (US); Rajagopal, Deven, Atlanta, GA 30327 (US)
(74) Representative: Bockhorni & Brüntjen Partnerschaft Patentanwälte mbB
(86) International application number: PCT/US2021/026580
(87) International publication number: WO 2021/211375

(56) References cited:
- US-A1- 2003 143 636
- US-A1- 2011 029 488
- US-A1- 2011 029 488
- US-A1- 2014 323 347
- US-A1- 2015 102 208
- US-A1- 2015 227 945
- US-B1- 7 128 258
- ADAM WOLISZ: "A Fully Distributed, Privacy Respecting Approach for Back-tracking of Potentially Infectious Contacts", ARXIV.ORG, CORNELL UNIVERSITY LIBRARY, 201 OLIN LIBRARY CORNELL UNIVERSITY ITHACA, NY 14853, 31 March 2020 (2020-03-31), XP081635039
- PHELAN ALEXANDRA L: "COVID-19 immunity passports and vaccination certificates: scientific, equitable, and legal challenges", SAFETY SCIENCE, vol. 395, 23 May 2020 (2020-05-23), AMSTERDAM, NL, pages 1595 - 1598, XP093100274, ISSN: 0925-7535, DOI: doi.org/10.1016/ S0140-6736(20)31034-5
- ANONYMOUS: "Security Token", WIKIPEDIA, 18 March 2020 (2020-03-18), pages 1 - 4, XP093100738, Retrieved from the Internet <URL:https://en.wikipedia.org/w/index.php?title=Security_token&oldid=946124304> [retrieved on 20231113]
- ANONYMOUS: "Biometrics", WIKIPEDIA, 30 June 2015 (2015-06-30), pages 1 - 7, XP055738890, Retrieved from the Internet <URL:https://en.wikipedia.org/w/index.php?title=Biometrics&oldid=666760290> [retrieved on 20201012]

## Description

This application claims the benefit of U.S. Provisional Patent Application Serial No. 63/009,133, filed April 13, 2020, the entirety of which is incorporated by reference herein.

### FIELD OF THE INVENTION

Embodiments of the present invention are generally related to providing a selectively accessible database that allows patients, doctors, or third parties to access predetermined medical records. In one embodiment, access to medical records is used to verify a user, e.g., a patient, has immunity to one or more pathogens.

### BACKGROUND OF THE INVENTION

Coronaviruses are well-known and are endemic in humans and animals. Most coronaviruses, such as those that cause the common cold, are minor, but some strains can cause severe illness. In particular, SARS-Co-V caused the SARS pandemic in 2003, and in 2019 a new strain of the coronavirus (SARS-CoV-2) was identified and quickly infected large portions of populations of many countries, causing the coronavirus disease 2019 (COVID-19) pandemic. The COVID-19 pandemic is expected to continue and will likely cause many deaths until vaccines or efficient treatment protocols are tested and implemented.

Because of its ease of transmission, including from asymptomatic carriers, countries affected by COVID-19 take steps to curb infection rates. These steps include requesting/requiring social distancing, wherein individuals are asked to remain a predetermined distance from each other; shelter-in-place orders whereby bars/restaurants and other businesses deemed nonessential are closed; reminding individuals not to touch their faces; reminding individuals to wash their hands consistently; wearing masks in public; etc.

Business and school closings adversely affect the economy and parents/' 'children's mental health. More importantly, the virulent nature and manifest symptoms caused by some viruses also take a tremendous toll on healthcare workers, often overwhelming the healthcare system's capacity. More specifically, although the COVID-19 virus, for example, causes many patients to exhibit mild flu-like symptoms, some patients, particularly the elderly or those with co-morbidities, require enhanced medical care that may call for the use of ventilators and other breathing and patient monitoring equipment. The machines and other devices required by some patients, in turn, increase the demand for skilled individuals to treat patients. Accordingly, one major issue identified during the treatment of the COVID-19 virus is that hospitals can be quickly overwhelmed with patients, resulting in longer hospital stays or increased mortality and limited care for patients with other conditions that require hospitalization.

Patients afflicted by COVID-19, like those afflicted by other diseases caused by coronaviruses and other viruses, will naturally create antibodies to the pathogen. These antibodies give the patient some degree of immunity for a predetermined time. More importantly, the antibodies can be taken from the formerly-infected patient and be used to create a serum for retreating currently infected patients. Formerly infected individuals are also unlikely to transmit the virus to others after a predetermined time post-infection. The presence of antibodies also would allow the patient to be "cleared" to work in jobs that formerly were off-limits as the chances of infection are high or to patronize any business (e.g., restaurants, bars, movie theaters, stores) that might otherwise be off-limits. For example, one of ordinary skill in the art will appreciate that healthcare workers are extremely vulnerable to be affected by many viruses, especially COVID-19, and it would be desirable to employ previously-infected or vaccinated individuals to maintain the needed hospital workforce. Business owners, such as those in the hotel, entertainment, bar, restaurant, etc., businesses, would also be desirous of hiring individuals that will not transmit the virus to customers and might desire to be open to immune customers.

It is foreseen that state, local, and foreign governments may mandate verification systems as a form of "immunity passport" to allow individuals to leave their homes, to gather in large groups, forgo mask-wearing, forgo social distancing rules, to work in certain industries, etc. Indeed, the U.S. government imposed strict vaccination guidelines to address a smallpox epidemic occurring in the early 1900s, so it reasonable to believe that the COVID-19 pandemic will force (or continue to force) entities to restrict civil liberties for the greater good of preserving life. It is also foreseen that individuals may be more willing to give up their civil liberties to be tracked with their mobile phone, randomly tested, etc. Notwithstanding any mandate, it is also foreseen that individuals may be willing to have their immunity status recorded in a voluntary database if it facilitates their ability to work, travel, and/or frequent businesses or other public places.

The following disclosure describes systems and methods for storing data related to pathogen exposure or vaccination. The data can be later used to verify immunity or provide a real-time certification that the patient is free of the pathogen and, thus, can perform certain tasks, be employed in certain locations, etc.

US 2015/0227945 A1 discloses a system to store, manage and maintain an individual's immunoprofile in a data base, which system is to be used to grant access to designed locations. While uploading the individual's immunoprofile into the database a secure log in process is used. Thereby, a general digital healthcare database is created, wherein the history of a patient's vaccination is stored in the database. The healthcare professional or the patient himself may check and verify whether the immunoprofile is complete without any gaps in protection.

US 2011/029488 A1 discloses a system for collecting and storing immunization and disease data on a database. The purpose of the system is to reduce the system of fragmented vaccine administration providers and points of service. In this context, an immunization mobile station is provided with a secure access system and equipped for storing the relevant data of the users. Inter alia, the potential side effects of a vaccination are stored. If, e.g., a temperature above the average for patients on day three after a vaccination is measured, an alarm is given to indicate that a referral to a clinician is recommended.

### SUMMARY OF THE INVENTION

The invention is defined by the subject matter of the independent claims.

It is one aspect of some embodiments of the present invention to provide a system for storing patient-specific data that is accessible by the patient, physician, or a third party upon request. The patient-specific data stored in one embodiment of the present invention is very top-level - 1) last name, 2) access code, 3) if the patient was exposed to a pathogen, and when exposure occurred, 4) if the patient was vaccinated against the pathogen, 5) whether the patient has been tested for antibodies. It is contemplated that the information obtained and stored is minimal such that compliance with data security rules, e.g., such as the California Consumer Privacy Act (CCPA) and European Union's General Data Protection Regulation (GDPR), are met. In some embodiments, however, the "owner" of the information, such as the patient, may opt in to provide more information, such as their complete health records and/or biometric information. This enhanced data may be included with the minimal data needed for embodiments of the present invention to function or accessed upon the input of additional patient-identifying codes or passwords to provide a heightened security level. As one of ordinary skill in the art will appreciate, dual-factor authentication or biometric data (e.g., facial recognition, fingerprints, retinal scans, etc.) may be used. The end-user may also choose to indicate their willingness to be a serum donor if needed so their antibodies can be gathered, opt-in for some notifications (some notifications would be mandatory - the vaccine you took was ineffective or corrupted), opt-in for mobile phone tracking, etc.

In operation, a doctor, healthcare worker, or certified testing facility verifies the patient has been exposed to a pathogen and enters that information into the database. The date of exposure can also be added such that the patient may be contagious can be calculated. For example, some infected patients are contagious at the beginning of exposure, wherein their chances of infecting others with the pathogen decrease over time, sometimes days/weeks after the patient has exhibited symptoms. Maintaining the date of first exposure or symptom will allow the healthcare professional to assess a date whern patient is no longer at risk for contaminating others. The database may also include vaccination dates, which could also include a future date that indicates the patient is no longer at risk for contaminating others. Finally, to ensure the information is sound, the doctor, for example, may be asked to include identifying information, such as their DEA number, NPI number, or other practice-specific information. Other healthcare workers (e.g., pharmacist, nurse, etc.) or testing centers (e.g., pharmacy, hospital, or ambulatory testing centers) may also be asked to provide specific identifying information. The testing lab associated with an antibody or PCR test, the vaccine lot number, etc. may also be stored in the database and accessed if it is later discovered that the testing procedures were incorrect, the vaccine lot was compromised, etc.

It is another aspect of some embodiments of the present invention to allow for selective access of data gathered to verify patient immunity. Here, the patient/healthcare professional/third-party can securely access the database through an electrical device, such as a home computer or mobile phone. In the operation of one embodiment, the end-user, the patient in this example, upon prompting by a third party, such as a potential employer, hospital system, airline, cruise ship, restaurant, theater, bar, state or federal health department, etc., can access the database real-time to provide a certification of prior exposure/immunity. The prompt for information may require a digital "handshake" wherein the user provides a portion of a secure code and another individual having access to the information, for example, the physician, application developer, another family member, etc., must input another piece of the code (e.g., dual-factor or better access protocols). Certification can be then delivered to the interested party, in one embodiment, by way of a secure message to a mobile device. Access to the database may require certain biometric information such as fingerprint or facial recognition to confirm that the individual holding the mobile phone, for example, is the individual whose data has been gathered, which will prevent fraud. Indeed, the third-party may also employ fraud-prevention tools, such as state or federal ID databases, to confirm the person touting their immunity status is the person providing the certificate.

Encryption may also be used. For example, in one embodiment, the user accesses database software by way of a computer or mobile application with a login name and password (dual-factor identification methods, biometrics, etc., could also be used) and, upon request, the database software generates a digital certificate, which may include a UPC code, a QR Code, or any other encrypted code. The digital certificate is then presented to a scanner to verify the user's health status.

It is another aspect of some embodiments of the present invention to use existing functionality provided by a user's mobile phone to actively communicate a patient's prior exposure, immunity, or non-exposure to the pathogen. For example, many mobile phones employ tools that allow individuals to find their friends or employ Bluetooth functionality that allows mobile phones to communicate with other mobile phones and devices a predetermined distance away. This functionality can be used to create a notice zone around the user's phone. In operation, a user previously exposed to the pathogen would broadcast that fact through their phone using, for example, Bluetooth, or via other signals that a mobile phone or other mobile device could conceivably generate (e.g., cellular, infra- or ultrasound waves). Any individual having access to an application associated with the contemplated database would access an "all clear" or "immune" signal and therefore appreciate why the person is not wearing a mask, not practicing social distancing, or working in a hospital or restaurant. Conversely, those who have not been exposed or those predisposed for re-exposure could broadcast that fact, wherein individuals would be more mindful to give that person a wider berth, for example.

The signal continuously broadcast or selectively sent by the user's mobile phone indicating immunity could be accessed by external sensors provided in bars, restaurants, movie theaters, hospitals, police stations, places of work or worship, etc., wherein access is only granted if the individual's phone triggers the sensor to indicate immunity. The contemplated sensor may be incorporated into a lock. Anti-fraud protocols can also be employed to ensure the person holding the mobile device is the actual person who is immune. These "safe spaces" protocols can be used to open restaurants, theaters, cruise ships, planes, trains, sections of hospitals, etc., to previously infected or vaccinated individuals. Conversely, other businesses would be reserved for those that have not been exposed to the pathogen. This separation can be used to help contain or monitor the spread of the pathogen. Ideally, all individuals carrying a mobile device will have access to a computer or mobile device would subscribe. For example, subscribers could permit their mobile devices to be tracked such that if some other subscriber (who may be unknown to them) later reported exposure and a probable date of exposure, the subscriber would be notified that their phone was in close proximity to or in the same building as a phone belonging to a person that has been exposed to a pathogen within a date window of highest contamination probability. The subscriber could then be directed to the nearest testing facility so they can be tested and, if infected, self-quarantine during the time they are the most contagious, which should drastically reduce infection rates.

It is also one aspect of one embodiment of the present invention provides a system that allows for individuals who have been affected and are now "safe" to work to provide their willingness to work and/or digital resume so that businesses needing previously infected workers to quickly identify a potential workforce. This aspect of some embodiments of the present invention may also allow for the potential employer to quickly review the immunity certificate described above.

As briefly mentioned above, some embodiments of the present invention provide the ability for notifications to be sent to subscribers of the app-based system that communicates with the database. These notifications can provide information regarding the possible loss of immunity due to time from exposure, an inadequate response to vaccine, testing error, or new data suggesting inaccuracy of a previously approved test. Again, such loss of immunity could be broadcast to other subscribers (friends/strangers), notifying them that they may be exposed because of the patient's loss of immunity. The notifications may also include reminders to receive a booster vaccine if necessary. The notifications may also include reminders to receive other vaccines for the flu, pneumonia, shingles, etc. The notifications may be linked to the patient's physical location. For example, if the patient is near a blood bank that is harvesting antibodies, and the patient had previously indicated a willingness to provide such antibodies, they would receive a notification that they are a predetermined distance from the blood bank with directions included in the notification. A prompt may also be provided that allows the user to make an appointment to receive a vaccine.

The data gathered by the contemplated systems may be used by health care professionals or public health organizations (e.g., CDC, WHO, etc.) to calculate rates of infection, recovery rates, movement of patients (if they have allowed or have been mandated to allow their mobile device to be tracked) to track possible exposure/immunity to facilitate, as examples, epidemiological research and public health interventions. In some instances, demographic data such as race, national origin, residence information, etc. may be gathered to assess whether a particular pathogen has an affinity to a certain group.

The mobile application contemplated by some embodiments of the present invention may also employ tools to notify individuals that it is safe to go to certain locations, such as grocery stores that have recently been opened and disinfected. The system may also provide notifications that the store is only open to those that have been infected, so they don't have to worry about infecting others. Of course, notifications can also include reminders to wash your hands, wash your mask, indicate that your mobile phone is less than a predetermined distance from another's mobile phone, etc. Although mobile phones have been mentioned herein, wearable devices, such as "smart" devices (e.g., smartwatches, fitness trackers, etc.) can perform the functionality described herein.

Embodiments of the present invention address the problems articulated above by providing a safe and effective way to verify that one is immune to a virus either by prior exposure or vaccination. The certification can be generated in real time and, thus, instantly verified. Again, in one embodiment, the verification is sent to a ' 'user's mobile device such as an Apple^{©} watch or smartphone. In other instances, the system generates a physical certification of prior exposure and immunity. Ideally, a real-time solution is provided, such that when queried by government officials, for example, a user can quickly prove they are immune.

The contemplated immunity inquiry may be initiated by a code produced by a third party coupled with a code produced by the user. For example, national or state border patrol authorities can provide a QR request code to a visitor requesting entry. The user would scan the QR code and input a " key." The system would read the QR code coupled with the user key and generate a secure code or authorization to generate a certificate that verifies immunity. The key may be comprised of identifying information such as a retinal scan, facial recognition, a fingerprint, or a unique useridentified pin number or password, which ensures a proper "handshake." The certificate of immunity is forwarded to the ' 'user's mobile device and/or the requester. Again, this real-time solution may be required during pandemics where pathogens spread very quickly.

These and other aspects and advantages will be apparent from the disclosure described herein. Further, the above-described embodiments, aspects, objectives, and configurations are neither complete nor exhaustive. As will be appreciated, other embodiments of the invention are possible using, alone or in combination, one or more of the features set forth above or described below. Additional aspects of the present invention will become more readily apparent from the Detailed Description, particularly when taken together with the drawings.

The above-described benefits, embodiments, and/or characterizations are not necessarily complete or exhaustive, and in particular, as to the patentable subject matter disclosed herein. Other benefits, embodiments, and/or characterizations of the present invention are possible utilizing, alone or in combination, as set forth above and/or described in the accompanying figures and/or in the description hereinbelow.

The phrases "at least one," "one or more," and "and/or," as used herein, are open-ended expressions that are both conjunctive and disjunctive in operation. For example, each of the expressions "at least one of A, B, and C," "at least one of A, B, or C," "one or more of A, B, and C," "one or more of A, B, or C," and "A, B, and/or C" means A alone, B alone, C alone, A and B together, A and C together, B and C together, or A, B, and C together.

The term "automatic" and variations thereof, as used herein, refers to any process or operation done without material human input when the process or operation is performed. However, a process or operation can be automatic, even though performance of the process or operation uses material or immaterial human input, if the input is received before performance of the process or operation. Human input is deemed to be material if such input influences how the process or operation will be performed. Human input that consents to the performance of the process or operation is not deemed to be "material".

The term "computer-readable medium", as used herein, refers to any tangible storage and/or transmission medium that participate in providing instructions to a processor for execution. Such a medium may take many forms, including but not limited to, non-volatile media, volatile media, and transmission media. Non-volatile media includes, for example, NVRAM, or magnetic or optical disks. Volatile media includes dynamic memory, such as main memory. Common forms of computer-readable media include, for example, a floppy disk, a flexible disk, hard disk, magnetic tape, or any other magnetic medium, magneto-optical medium, a CD-ROM, any other optical medium, punch cards, paper tape, any other physical medium with patterns of holes, a RAM, a PROM, and EPROM, a FLASH-EPROM, a solid state medium like a memory card, any other memory chip or cartridge, a carrier wave as described hereinafter, or any other medium from which a computer can read. A digital file attachment to e-mail or other self-contained information archive or set of archives is considered a distribution medium equivalent to a tangible storage medium. When the computer-readable media is configured as a database, it is to be understood that the database may be any type of database, such as relational, hierarchical, object-oriented, and/or the like. Accordingly, the disclosure is considered to include a tangible storage medium or distribution medium and prior art-recognized equivalents and successor media, in which the software implementations of the present disclosure are stored.

The terms "determine", "calculate" and "compute," and variations thereof, as used herein, are used interchangeably and include any type of methodology, process, mathematical operation or technique.

The term "module", as used herein, refers to any known or later developed hardware, software, firmware, artificial intelligence, fuzzy logic, or combination of hardware and software that is capable of performing the functionality associated with that element.

Unless otherwise indicated, all numbers expressing quantities, dimensions, conditions, and so forth used in the specification and drawing figures are to be understood as being approximations that may be modified in all instances as required for a particular application of the novel assembly and method described herein.

The term "a" or "an" entity, as used herein, refers to one or more of that entity. As such, the terms "a" (or "an"), "one or more" and "at least one" can be used interchangeably herein.

The use of "including," "comprising," or "having" and variations thereof herein is meant to encompass the items listed thereafter and equivalents thereof as well as additional items. Accordingly, the terms "including," "comprising," or "having" and variations thereof can be used interchangeably herein.

It shall be understood that the term "means" as used herein shall be given its broadest possible interpretation per 35 U.S.C., Section 112(f). Accordingly, a claim incorporating the term "means" shall cover all structures, materials, or acts set forth herein, and all of the equivalents thereof. Further, the structures, materials, or acts and the equivalents thereof shall include all those described in the Summary, Brief Description of the Drawings, Detailed Description and in the appended drawing figures.

### BRIEF DESCRIPTION OF THE DRAWINGS

The accompanying drawings, which are incorporated in and constitute a part of the specification, illustrate embodiments of the invention and together with the general description of the invention given above and the detailed description of the drawings given below, serve to explain the principles of these inventions.
Fig. 1 is a process flow of a method of one embodiment of the present invention.
Fig. 2 is a process flow of a method of another embodiment of the present invention.
Fig. 3 is an example communications/data processing network system that may be used in conjunction with embodiments of the present invention.
Fig. 4 is an example computer system that may be used in conjunction with embodiments of the present invention.

It should be understood that the drawings are not necessarily to scale. In certain instances, details that are not necessary for an understanding of the invention or that render other details difficult to perceive may have been omitted. It should be understood, of course, that the invention is not necessarily limited to the particular embodiments illustrated herein.

### DETAILED DESCRIPTION

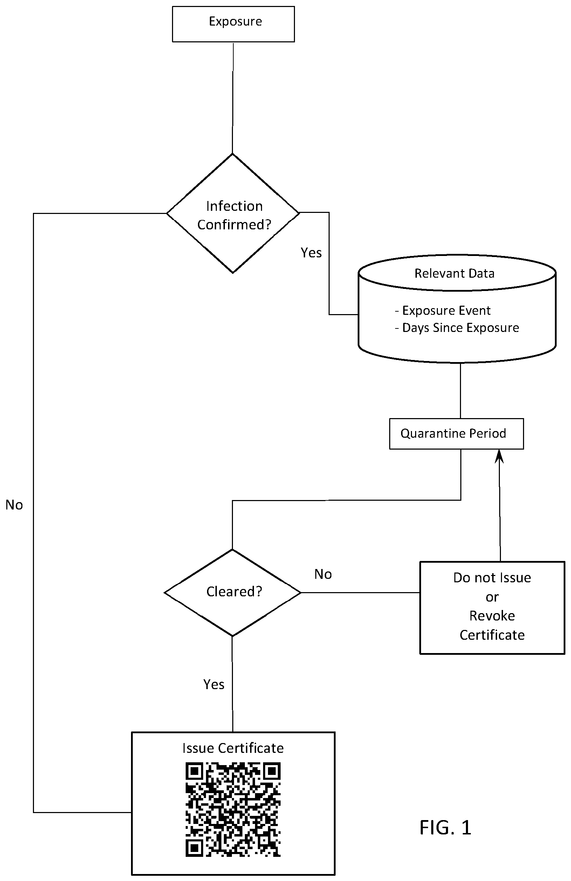

Fig. 1 shows system implementation of one embodiment of the present invention. After virus exposure, a user obtains confirmation of infection either by physical examination by a healthcare professional or, more commonly, from a positive test. One of ordinary skill in the art will appreciate that results of such tests may take hours to days. If infection is not confirmed, a certification is issued to the user. The certificate could be in the form of a QR code issued to user's mobile device, which may be scannable by government authorities, employers, guards, airline personnel, etc. Of course, other certificate forms, digital or physical, may be issued without departing from the scope of the invention.

If an infection is confirmed, relevant data is gathered, which will help healthcare providers and users appreciate how and when the exposure occurred. This data may be important to curb additional pathogen spread. For example, events that may have caused the exposure, the date of possible exposure, and symptoms that predicated the visit to the healthcare provider may be logged. This information is used to set a quarantine period so the condition can run its course. It is important to note that infection confirmation is not required. More specifically, if exposure is likely, healthcare experts or state protocols may treat the user as being exposed, wherein relevant data is collected.

After the quarantine period has passed, a healthcare provider will "clear" the patient, which may entail issuing an immunity certificate and/or patient-specific release code that is entered into the contemplated system and used to selectively generate an immunity certificate. The release code may be associated with a physician-specific identifier, patient-specific identifier, or combination thereof. If the physician or other qualified healthcare professional does not believe the patient is immune because of subsequent tests, or lingering symptoms, the certificate is not issued and the patient is returned to quarantine for a predetermined time. This clearance step can be conducted multiple times, wherein previously-issued certificates may be revoked if needed.

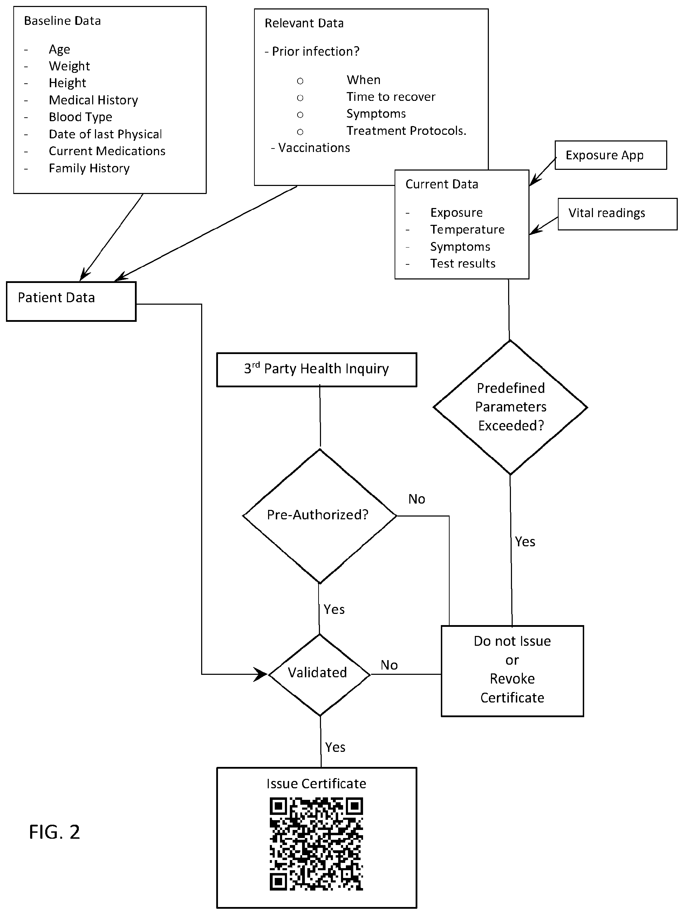

Fig. 2 shows the method of another embodiment of the present invention. Here, patient data is stored in a database that includes patient baseline data. The baseline data is associated with traditional medical records and may include age, weight, ethnic background, medical history, blood type, family history, etc. Coupled with the baseline data is data more relevant to the pathogen of concern. This "relevant data" may include prior infections, date of prior infection, recovery time, symptoms, treatment protocols, etc. It may also include vaccinations and immunity data because immunity may not last for more than a predetermined time.

The relevant data is also associated with more current, real-time data similar to what was described in Fig. 1. For example, "current data" is related to the patient's temperature, recent test results, etc. This sub-database may receive information from third-party exposure apps. Vital readings from the user's smart device or wearable may also be fed into the current database. The vital data may include temperature, heart rate, etc. In one embodiment, user temperature is obtained by the methods described in U.S. Patent No. 6,292,685 and 7,787,938, which are incorporated by reference herein. The current database may also draw information from social media websites, wherein posts by the user's friends are monitored to identify instances of exposure, which may be communicated to the patient. For example, if a user's friend indicates they were at a party and now have contracted COVID-19, such information is forwarded to the user or stored, wherein the user could be queried as to their whereabouts during the party. The user's internet search history could also be monitored to assess whether they are searching for information regarding symptoms of a pathogen of concern.

Upon an immunization or immunity inquiry from a third-party, the system will assess whether such inquiry has been authorized. Prior authorization can come from the user or may be mandated. If the inquiry was not approved, the certification of immunity is not issued. If the inquiry comes from the user, no preauthorization to access patient data and immunity validation is needed.

Next, the patient's health is validated, which entails drawing information from the patient database and may include input by a healthcare professional. The healthcare professional's approval may have been pre-obtained if, for example, a prior certificate has been issued. In some instances, prior certificates may become "stale" after a predetermined amount of time, wherein healthcare professionals may need to reassess patient data to re-recommend the issuance of a certificate. If the patient's health is validated, a certificate is issued as described above.

However, if the patient's immunity status is not validated, issuance of a new certificate is denied or a previously-issued certificate is revoked. Certificate revocation also happens in one embodiment of the present invention if predetermined parameters are exceeded. For example, the current database is continually updated with the user's temperature. If the user's temperature exceeds a predetermined level, the currentlyissued certificate is revoked or flagged for further review. Certificate revocation may generate a notification to the user, the doctor, or other third parties, depending on user preferences. This will allow others that are part of the system to be quickly notified of possible exposure because of their friend's infection or reinfection.

As alluded to above, certificates can be temporal. Commonly, the certificate will sunset over time and expire. In another embodiment, the strength of the certificate may be apparent by a color code or other designation. For example, a green certificate would mean that the validation of immunity has occurred within a predetermined amount of time, e.g., six hours. The certificate color may change or fade over time based on various factors.

Embodiments of the present invention contemplate the issuance of notices when a certificate is issued, not issued, or revoked. Such information is sent to predefined interested parties. For example, the notification or certificate could be sent to a user's workplace or employment websites. The certificate can be used to unlock doors, wherein scanning of the certificate acts as a key.

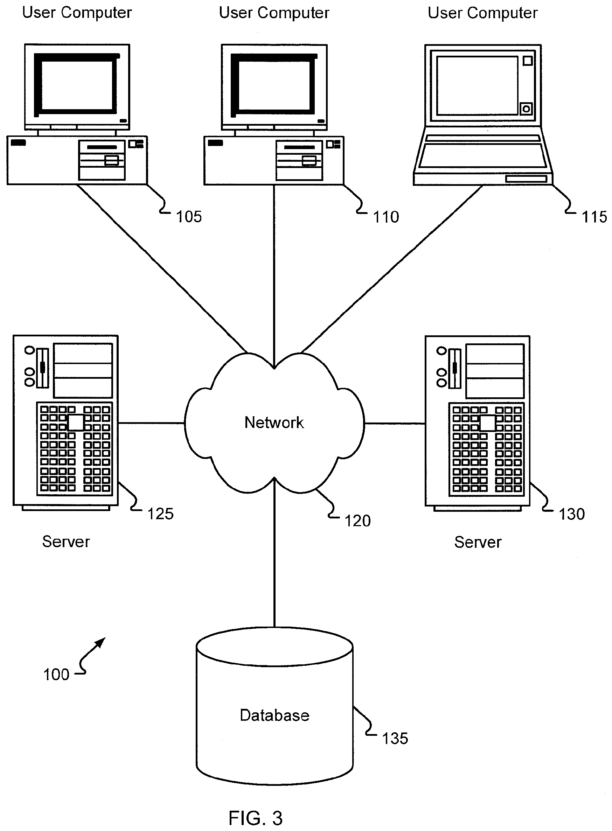

Referring to Fig. 3, an example network system is provided that may be used in connection with the classification systems and methods disclosed herein. More specifically, Fig. 3 illustrates a block diagram of a system 100 that may use a web service connector to integrate an application with a web service. The system 100 includes one or more user computers 105, 110, and 115. The user computers 105, 110, and 115 may be general-purpose personal computers (including, merely by way of example, personal computers and/or laptop computers running various versions of Microsoft Corp.'s Windows^{™} and/or Apple Corp.'s Macintosh^{™} operating systems) and/or workstation computers running any of a variety of commercially-available UNIX^{™} or UNIX-like operating systems. These user computers 105, 110, 115 may also have any of a variety of applications, including, for example, database client and/or server applications, and web browser applications. Alternatively, the user computers 105, 110, and 115 may be any other electronic device, such as a thin-client computer, Internet-enabled mobile telephone, and/or personal digital assistant, capable of communicating via a network (e.g., the network 120 described below) and/or displaying and navigating web pages or other types of electronic documents. Although the exemplary system 100 is shown with three user computers, any number of user computers may be supported.

System 100 further includes a network 120. The network 120 may be any type of network familiar to those skilled in the art that can support data communications using any of a variety of commercially-available protocols, including without limitation TCP/IP, SNA, IPX, AppleTalk, and the like. Merely by way of example, the network 120 may be a local area network ("LAN"), such as an Ethernet network, a Token-Ring network and/or the like; a wide-area network; a virtual network, including without limitation a virtual private network ("VPN"); the Internet; an intranet; an extranet; a public switched telephone network ("PSTN"); an infra-red network; a wireless network (e.g., a network operating under any of the IEEE 802.11 suite of protocols, the Bluetooth^{™} protocol known in the art, and/or any other wireless protocol); and/or any combination of these and/or other networks.

The system 100 may also include one or more server computers 125, 130. One server may be a web server 125, which may be used to process requests for web pages or other electronic documents from user computers 105, 110, and 120. The web server can be running an operating system including any of those discussed above, as well as any commercially-available server operating systems. The web server 125 can also run a variety of server applications, including HTTP servers, FTP servers, CGI servers, database servers, Java servers, and the like. In some instances, the web server 125 may publish operations available as one or more web services.

The system 100 may also include one or more file and/or application servers 130, which can, in addition to an operating system, include one or more applications accessible by a client running on one or more of the user computers 105, 110, 115. The server(s) 130 may be one or more general-purpose computers capable of executing programs or scripts in response to the user computers 105, 110 and 115. As one example, the server may execute one or more web applications. The web application may be implemented as one or more scripts or programs written in any programming language, such as JavaTM, C, C#^{™} or C++, and/or any scripting language, such as Perl, Python, or TCL, as well as combinations of any programming/scripting languages. The application server(s) 130 may also include database servers, including without limitation those commercially available from Oracle, Microsoft, SybaseTM, IBMTM and the like, which can process requests from database clients running on a user computer 105.

In some embodiments, an application server 130 may create web pages dynamically for displaying the development system. The web pages created by the web application server 130 may be forwarded to a user computer 105 via a web server 125. Similarly, the web server 125 may be able to receive web page requests, web services invocations, and/or input data from a user computer 105 and can forward the web page requests and/or input data to the web application server 130.

In further embodiments, the server 130 may function as a file server. Although for ease of description, Fig. 3 illustrates a separate web server 125 and file/application server 130, those skilled in the art will recognize that the functions described with respect to servers 125, 130 may be performed by a single server and/or a plurality of specialized servers, depending on implementation-specific needs and parameters.

The system 100 may also include a database 135. The database 135 may reside in a variety of locations. By way of example, database 135 may reside on a storage medium local to (and/or resident in) one or more of the computers 105, 110, 115, 125, 130. Alternatively, it may be remote from any or all of the computers 105, 110, 115, 125, 130, and in communication (e.g., via the network 120) with one or more of these. In a particular set of embodiments, the database 135 may reside in a storage-area network ("SAN") familiar to those skilled in the art. Similarly, any necessary files for performing the functions attributed to the computers 105, 110, 115, 125, 130 may be stored locally on the respective computer and/or remotely, as appropriate. In one set of embodiments, the database 135 may be a relational database, such as Oracle 10i^{™}, that is adapted to store, update, and retrieve data in response to SQL-formatted commands.

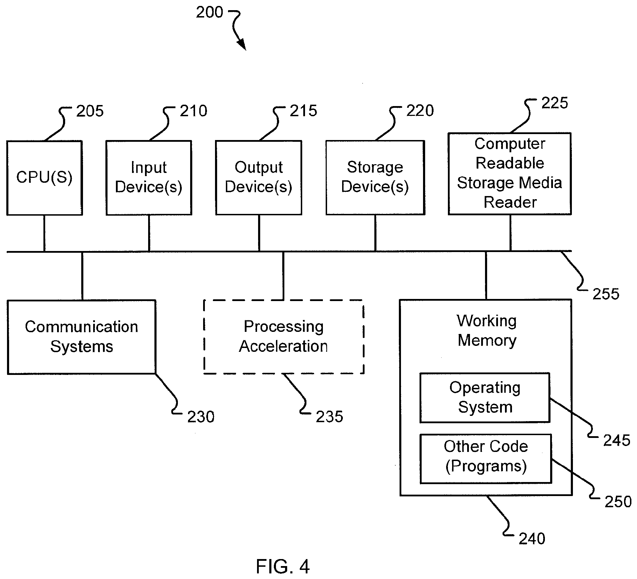

Referring to Fig. 4, an example computer system is provided that may be used in connection with the classification systems and methods disclosed herein. More specifically, Figure 2 illustrates one embodiment of a computer system 200 upon which a web service connector or components of a web service connector may be deployed or executed. The computer system 200 is shown comprising hardware elements that may be electrically coupled via a bus 255. The hardware elements may include one or more central processing units (CPUs) 205; one or more input devices 210 (e.g., a mouse, a keyboard, etc.); and one or more output devices 215 (e.g., a display device, a printer, etc.). The computer system 200 may also include one or more storage device 220. By way of example, storage device(s) 220 may be disk drives, optical storage devices, solid-state storage device such as a random access memory ("RAM") and/or a readonly memory ("ROM"), which can be programmable, flash-updateable and/or the like.

The computer system 200 may additionally include a computer-readable storage media reader 225; a communications system 230 (e.g., a modem, a network card (wireless or wired), an infra-red communication device, etc.); and working memory 240, which may include RAM and ROM devices as described above. In some embodiments, the computer system 200 may also include a processing acceleration unit 235, which can include a DSP, a special-purpose processor and/or the like.

The computer-readable storage media reader 225 can further be connected to a computer-readable storage medium, together (and, optionally, in combination with storage device(s) 220) comprehensively representing remote, local, fixed, and/or removable storage devices plus storage media for temporarily and/or more permanently containing computer-readable information. The communications system 230 may permit data to be exchanged with the network 220 and/or any other computer described above with respect to the system 200.

The computer system 200 may also comprise software elements, shown as being currently located within a working memory 240, including an operating system 245 and/or other code 250, such as program code implementing a web service connector or components of a web service connector. It should be appreciated that alternate embodiments of a computer system 200 may have numerous variations from that described above. For example, customized hardware might also be used and/or particular elements might be implemented in hardware, software (including portable software, such as applets), or both. Further, connection to other computing devices such as network input/output devices may be employed.

It should be appreciated that the methods described herein may be performed by hardware components or may be embodied in sequences of machine-executable instructions, which may be used to cause a machine, such as a general-purpose or special-purpose processor or logic circuits programmed with the instructions to perform the methods. These machine-executable instructions may be stored on one or more machine-readable mediums, such as CD-ROMs or other type of optical disks, floppy diskettes, ROMs, RAMs, EPROMs, EEPROMs, magnetic or optical cards, flash memory, or other types of machine-readable mediums suitable for storing electronic instructions. Alternatively, the methods may be performed by a combination of hardware and software.

Exemplary characteristics of embodiments of the present invention have been described. However, to avoid unnecessarily obscuring embodiments of the present invention, the preceding description may omit several known apparatus, methods, systems, structures, and/or devices one of ordinary skill in the art would understand are commonly included with the embodiments of the present invention. Such omissions are not to be construed as a limitation of the scope of the claimed invention. Specific details are set forth to provide an understanding of some embodiments of the present invention. It should, however, be appreciated that embodiments of the present invention may be practiced in a variety of ways beyond the specific detail set forth herein.

Modifications and alterations of the various embodiments of the present invention described herein will occur to those skilled in the art.

In the foregoing Detailed Description, various features of the invention are grouped together in one or more embodiments for the purpose of streamlining the disclosure. Further, the embodiments of the present invention described herein include components, methods, processes, systems, and/or apparatus substantially as depicted and described herein, including various sub-combinations and subsets thereof. Accordingly, one of skill in the art will appreciate that would be possible to provide for some features of the embodiments of the present invention without providing others. Stated differently, any one or more of the aspects, features, elements, means, or embodiments as disclosed herein may be combined with any one or more other aspects, features, elements, means, or embodiments as disclosed herein.

## Claims

1. A computer-implemented method for verifying patient immunity to at least one pathogen, comprising:
obtaining, by a patient information module, patient-specific data, comprising at least one patient identifying characteristic;
obtaining, by a pathogen exposure module, exposure data related to patient exposure to the at least one pathogen;
gathering data related to patient infection;
verifying, by a verification module, patient immunity;
creating a digital record of patient immunity;
adding a predefined validation code by a healthcare professional to at least one of the patient-specific data, exposure data, and digital record of patient immunity;
forwarding the patient-specific data, exposure data, and digital record of patient immunity to a database;
selectively accessing the database upon compliance with at least one security protocol;
generating, by a certificate generation module, a digital certificate related to the patient's immunity to the at least one pathogen;
wherein the digital certificate can only be generated within a predetermined time window, and
wherein the body temperature is measured and the digital certificate is revoked if the patient's body temperature is elevated above a predetermined degree.

2. The method of claim 1, wherein data related to patient infection comprises exposure data and/or vaccination data.

3. The method of claim 1 or 2, wherein the validation code includes data specific to the healthcare professional.

4. The method of any one of the preceding claims, wherein the digital certificate is altered as a function of the time between exposure to the at least one pathogen and issuance of the digital certificate.

5. The method of any one of the preceding claims, wherein the exposure to the at least one pathogen is detected through a sensor integrated into an insulin pump or sensor.

6. The method of any one of the preceding claims, wherein the at least one security protocol comprises requiring a patient code before the digital certificate is issued.

7. The method of claim 6, wherein the patient code must be coupled with a randomly-generated requestor code before the digital certificate is issued.

8. The method of claim 6 or 7, wherein the patient code is biometric.

9. The method of claim 1, wherein measurement of body temperature comprises:
scanning a temperature detector across an artery;
providing a peak temperature reading from plural readings obtained during scanning, wherein the temperature detector comprises a radiation sensor that views a target surface area;
further comprising computing an internal body temperature as a function of ambient temperature and the peak temperature reading; and
wherein the function includes a weighted difference of surface temperature and ambient temperature, the weighting including an approximation of h/pc at an artery where h is a heat transfer coefficient between a target surface and ambient, p is perfusion rate, and c is blood specific heat.

10. The method of claim 8 or 9 further comprising issuing a notice that a certificate has been issued or revoked.

11. A system for verifying patient immunity to at least one pathogen, comprising:
a database comprising:
at least one processor,
a patient information module comprised of at least one processor configured to selectively receive at least one patient identifying characteristic;
a pathogen exposure module comprised of at least one processor configured to receive data related to patient exposure to the at least one pathogen, the pathogen exposure module also configured to communicate with the patient information module;
a verification module that receives input from a healthcare professional regarding patient immunity to the pathogen;
a certificate generation module that communicates with the database upon compliance with predetermined security protocols, the certificate generation module issues a digital certificate;
a notification module that communicates information from the database to an end user;
an input device that communicates with database;
a monitoring module configured to communicate with at least one of the patient information module and the pathogen exposure module; and
wherein the monitoring module is configured to communicate with a temperature monitoring device,
wherein the digital certificate is revoked if the patient's body temperature is elevated above a predetermined degree.

12. The system of claim 11 wherein the monitoring module and/or certificate generation module is in communication with a door lock.

13. The system of claim 11 or 12, wherein the monitoring module is configured to communicate with a GPS device.

## Patentansprüche

1. Computerimplementiertes Verfahren zum Verifizieren einer Patientenimmunität gegenüber mindestens einem Krankheitserreger, umfassend:
Erhalten, durch ein Patienteninformationsmodul, patientenspezifischer Daten, die mindestens ein patientenidentifizierendes Merkmal umfassen;
Erhalten, durch ein Krankheitserreger-Expositionsmodul, von Expositionsdaten bezüglich einer Patientenexposition gegenüber dem mindestens einen Krankheitserreger;
Sammeln von Daten bezüglich einer Patienteninfektion;
Verifizieren, durch ein Verifizierungsmodul, der Patientenimmunität;
Erstellen einer digitalen Akte der Patientenimmunität;
Hinzufügen eines vordefinierten Validierungscodes durch eine medizinische Fachkraft zu mindestens einem der patientenspezifischen Daten, der Expositionsdaten und der digitalen Akte der Patientenimmunität;
Weiterleiten der patientenspezifischen Daten, der Expositionsdaten und der digitalen Akte der Patientenimmunität an eine Datenbank;
selektives Zugreifen auf die Datenbank bei Einhaltung mindestens eines Sicherheitsprotokolls;
Generieren, durch ein Zertifikatgenerierungsmodul, eines digitalen Zertifikats bezüglich der Immunität des Patienten gegenüber dem mindestens einen Krankheitserreger;
wobei das digitale Zertifikat nur innerhalb eines zuvor festgelegten Zeitfensters generiert werden kann, und
wobei die Körpertemperatur gemessen wird und das digitale Zertifikat widerrufen wird, falls die Körpertemperatur des Patienten über einen zuvor festgelegten Grad hinaus ansteigt.

2. Verfahren nach Anspruch 1, wobei Daten bezüglich einer Patienteninfektion Expositionsdaten und/oder Impfdaten umfassen.

3. Verfahren nach Anspruch 1 oder 2, wobei der Validierungscode Daten enthält, die für die medizinische Fachkraft spezifisch sind.

4. Verfahren nach einem der vorangehenden Ansprüche, wobei das digitale Zertifikat in Abhängigkeit von der Zeit zwischen der Exposition gegenüber dem mindestens einen Krankheitserreger und der Ausstellung des digitalen Zertifikats geändert wird.

5. Verfahren nach einem der vorangehenden Ansprüche, wobei die Exposition gegenüber dem mindestens einen Krankheitserreger durch einen in eine Insulinpumpe oder einen Insulinsensor integrierten Sensor detektiert wird.

6. Verfahren nach einem der vorangehenden Ansprüche, wobei das mindestens eine Sicherheitsprotokoll umfasst, einen Patientencode anzufordern, bevor das digitale Zertifikat ausgestellt wird.

7. Verfahren nach Anspruch 6, wobei der Patientencode mit einem nach dem Zufallsprinzip generierten Anforderercode gekoppelt werden muss, bevor das digitale Zertifikat ausgestellt wird.

8. Verfahren nach Anspruch 6 oder 7, wobei der Patientencode biometrisch ist.

9. Verfahren nach Anspruch 1, wobei die Messung der Körpertemperatur umfasst:
Scannen eines Temperaturdetektors an einer Arterie;
Bereitstellen eines Spitzentemperaturmesswertes aus mehreren während des Scannens erhaltenen Messwerten, wobei der Temperaturdetektor einen Strahlungssensor umfasst, der einen Zieloberflächenbereich beobachtet;
des Weiteren umfassend das Berechnen einer inneren Körpertemperatur als eine Funktion der Umgebungstemperatur und des Spitzentemperaturmesswertes; und
wobei die Funktion eine gewichtete Differenz von Oberflächentemperatur und Umgebungstemperatur enthält, wobei das Gewichten eine Annäherung von h/pc an einer Arterie umfasst, wobei h ein Wärmeübertragungskoeffizient zwischen einer Zieloberfläche und der Umgebung ist, p die Perfusionsrate ist und c eine blutspezifische Wärme ist.

10. Verfahren nach Anspruch 8 oder 9, des Weiteren umfassend das Ausgeben einer Mitteilung, dass ein Zertifikat ausgestellt oder widerrufen wurde.

11. System zum Verifizieren einer Patientenimmunität gegenüber mindestens einem Krankheitserreger, umfassend:
eine Datenbank, umfassend:
mindestens einen Prozessor,
ein Patienteninformationsmodul, das mindestens einen Prozessor umfasst, der dazu eingerichtet ist, selektiv mindestens ein patientenidentifizierendes Merkmal zu empfangen;
ein Krankheitserreger-Expositionsmodul, das mindestens einen Prozessor umfasst, der dazu eingerichtet ist, Daten bezüglich einer Patientenexposition gegenüber dem mindestens einen Krankheitserreger zu empfangen, wobei das Krankheitserreger-Expositionsmodul außerdem dazu eingerichtet ist, mit dem Patienteninformationsmodul zu kommunizieren;
ein Verifizierungsmodul, das von einer medizinischen Fachkraft Eingaben bezüglich der Patientenimmunität gegenüber dem Krankheitserreger empfängt;
ein Zertifikatgenerierungsmodul, das bei Einhaltung zuvor festgelegter Sicherheitsprotokolle mit der Datenbank kommuniziert, wobei das Zertifikatgenerierungsmodul ein digitales Zertifikat ausstellt;
ein Benachrichtigungsmodul, das Informationen von der Datenbank an einen Endnutzer übermittelt;
eine Eingabevorrichtung, die mit der Datenbank kommuniziert;
ein Überwachungsmodul, das dazu eingerichtet ist, mit mindestens einem des Patienteninformationsmoduls und des Krankheitserreger-Expositionsmoduls zu kommunizieren; und
wobei das Überwachungsmodul für eine Kommunikation mit einer Temperaturüberwachungsvorrichtung eingerichtet ist,
wobei das digitale Zertifikat widerrufen wird, falls die Körpertemperatur des Patienten über einen zuvor festgelegten Grad hinaus ansteigt.

12. System nach Anspruch 11, wobei das Überwachungsmodul und/oder das Zertifikatgenerierungsmodul in Kommunikation mit einem Türschloss stehen.

13. System nach Anspruch 11 oder 12, wobei das Überwachungsmodul dazu eingerichtet ist, mit einer GPS-Vorrichtung zu kommunizieren.

## Revendications

1. Procédé mis en œuvre par ordinateur permettant de vérifier l'immunité d'un patient à l'égard d'au moins un agent pathogène, comprenant :
l'obtention, par un module d'information sur le patient, de données spécifiques au patient, comprenant au moins une caractéristique d'identification du patient ;
l'obtention, par un module d'exposition aux agents pathogènes, de données relatives à l'exposition du patient à l'au moins un agent pathogène ;
la collecte de données relatives à une infection du patient ;
la vérification, par un module de vérification, de l'immunité du patient ;
la création d'un dossier numérique d'immunité du patient ;
l'ajout d'un code de validation prédéfini par un professionnel de la santé à au moins l'un parmi les données spécifiques au patient, les données d'exposition, et le dossier numérique d'immunité du patient ;
la transmission des données spécifiques au patient, des données d'exposition, et du dossier numérique d'immunité du patient à une base de données ;
l'accès sélectif à la base de données en respectant au moins un protocole de sécurité ;
la génération, par un module de génération de certificats, d'un certificat numérique relatif à l'immunité du patient à l'égard de l'au moins un agent pathogène ;
dans lequel le certificat numérique ne peut être généré que dans une fenêtre temporelle prédéterminée, et
dans lequel la température corporelle est mesurée et le certificat numérique est révoqué si la température corporelle du patient est supérieure à un degré prédéterminé.

2. Procédé selon la revendication 1, dans lequel des données relatives à l'infection du patient comprennent des données d'exposition et/ou des données de vaccination.

3. Procédé selon la revendication 1 ou 2, dans lequel le code de validation comporte des données spécifiques au professionnel de la santé.

4. Procédé selon l'une quelconque des revendications précédentes, dans lequel le certificat numérique est modifié en fonction du temps écoulé entre l'exposition à au moins un agent pathogène et l'émission du certificat numérique.

5. Procédé selon l'une quelconque des revendications précédentes, dans lequel l'exposition à l'au moins un agent pathogène est détectée par un capteur intégré à une pompe à insuline ou un capteur.

6. Procédé selon l'une quelconque des revendications précédentes, dans lequel l'au moins un protocole de sécurité comprend le fait de demander un code patient avant l'émission du certificat numérique.

7. Procédé selon la revendication 6, dans lequel le code patient doit être associé à un code de demandeur généré de manière aléatoire avant que le certificat numérique ne soit délivré.

8. Procédé selon la revendication 6 ou 7, dans lequel le code patient est biométrique.

9. Procédé selon la revendication 1, dans lequel la mesure de la température corporelle comprend :
le balayage d'un détecteur de température sur une artère ;
la fourniture d'une lecture de température maximale à partir de plusieurs lectures obtenues pendant le balayage, dans lequel le détecteur de température comprend un capteur de rayonnement qui visualise une surface cible ;
comprenant en outre le calcul d'une température corporelle interne en fonction de la température ambiante et de la lecture de température maximale ; et
dans lequel la fonction inclut une différence pondérée entre la température de surface et la température ambiante, la pondération incluant une approximation de h/pc au niveau d'une artère où h est un coefficient de transfert de chaleur entre une surface cible et l'environnement, p est le taux de perfusion, et c est la chaleur spécifique du sang.

10. Procédé selon la revendication 8 ou 9, comprenant en outre l'émission d'un avis indiquant qu'un certificat a été émis ou révoqué.

11. Système permettant de vérifier l'immunité d'un patient à l'égard d'au moins un agent pathogène, comprenant :
une base de données comprenant :
au moins un processeur,
un module d'information sur le patient comprenant au moins un processeur configuré pour recevoir sélectivement au moins une caractéristique d'identification du patient ;
un module d'exposition aux agents pathogènes comprenant au moins un processeur configuré pour recevoir des données relatives à l'exposition du patient à l'au moins un agent pathogène, le module d'exposition aux agents pathogènes étant également configuré pour communiquer avec le module d'information sur le patient ;
un module de vérification qui reçoit des informations d'un professionnel de la santé concernant l'immunité du patient vis-à-vis de l'agent pathogène ;
un module de génération de certificats qui communique avec la base de données en respectant des protocoles de sécurité prédéterminés, le module de génération de certificats émet un certificat numérique ;
un module de notification qui communique les informations de la base de données à un utilisateur final ;
un dispositif d'entrée qui communique avec la base de données ;
un module de surveillance configuré pour communiquer avec au moins l'un du module d'information sur le patient et du module d'exposition aux agents pathogènes ; et
le module de surveillance est configuré pour communiquer avec un dispositif de surveillance de la température,
dans lequel le certificat numérique est révoqué si la température corporelle du patient est supérieure à un degré prédéterminé.

12. Système selon la revendication 11, dans lequel le module de surveillance et/ou le module de génération de certificats sont en communication avec une serrure de porte.

13. Système selon la revendication 11 ou 12, dans lequel le module de surveillance est configuré pour communiquer avec un dispositif GPS.
